# EUROPEAN PATENT APPLICATION

(11) **EP 3 135 234 A1**
(43) Date of publication of application: **01.03.2017**
(21) Application number: 16183420.5
(22) Date of filing: 09.08.2016
(51) Int. Cl.: A61B 17/80

(54) **A THREE-DIMENSIONAL FRACTURE FIXATION DEVICE FOR NERVE PROTECTION**

(30) Priority: 28.08.2015 US 201514839762
(71) Applicant: Loregen Biotechnology Corp., Taoyuan City 320 (TW)
(72) Inventor: Tan, Tai-Sheng, 40756 Taichung City (TW); Chung, Yih-Lin, 106 Taipei (TW); Hsiao, Tien-Mu, 302 Zhubei City, Hsinchu County (TW)
(74) Representative: Michalski Hüttermann & Partner Patentanwälte mbB

(57) **Abstract**

A three-dimensional fracture fixation device is provided for treating clavicle fracture with the intent to protect the supraclavicular nerve from later iatrogenic transsection injury that may occur when the fixation bone plate is removed. The essential components of the three-dimensional fracture fixation device include at least one fixation locking plate, a nerve protection scutum (shield), junctional hooks connecting the fixation locking plate and supporting the nerve protection scutum (shield), and bone screws, which are assembled to form a defined space to accommodate the supraclavicular nerve during open reduction and internal fixation.

## Description

### BACKGROUND OF THE INVENTION

### Field of the Invention

The invention relates to a three-dimensional fracture fixation device for nerve protection for such as clavicle fracture.

### Description of the Related Art

The classic paradigm teaches that clavicle fractures can be treated non-operatively even if both ends of the clavicle are widely separated (Canadian Orthopaedic Trauma Society. J Bone Joint Surg Am. 2007 Jan; 89(1):1-10.; Zlowodzki M, et al. J Orthop Trauma. 2005 Aug; 19(7):504-7.; Nordqvist A, et al. J Orthop Trauma. 1998 Nov-Dec; 12(8):572-6.). However, recent studies have shown a nonunion rate of 15% as well as a 31% unsatisfactory patient-oriented outcome under conservative treatment (Altamimi SA, et al. J Bone Joint Surg Am. 2008 Mar; 90 Suppl 2 Pt 1:1-8.). The management of clavicle fractures has changed with time. Primary fixation using bone plates has dramatically increased (Lee SK, et al. Orthopedics. 2013 Jun; 36(6):801-7.; Soha Sajid. et al. Int J Shoulder Surg. 2012 Oct-Dec; 6(4): 126-129.).

The supraclavicular nerve lies in close proximity to the clavicle (Tyler Nathe, et al. Clin Orthop Relat Res. 2011 Mar; 469(3): 890-894.). Although the supraclavicular nerve branches including the lateral, intermediate and medial groups can be carefully identified in the initial open reduction and bone plate fixation procedure, it is particularly vulnerable to later iatrogenic transsection during skin incision and tissue dissection for removal of the bone plate when clavicle fracture heals and the bone plate cause discomfort because the supraclavicular nerve is stretched by the surrounding scarring tissues or entrapped in the callous formation (Erdogan M, et al. Acta Chir Orthop Traumatol Cech. 2014;81(6):387-91.; Wang L, et al. Cutaneous hypoesthesia following plate fixation in clavicle fractures.; O'Neill K, et al. J Orthop Trauma. 2012 Jun;26(6):e63-5.; Wang k, et al. Injury. 2010 Oct; 41(10):1002-5.) The sensorial injury of the supraclavicular nerve results in cutaneous sensory loss over the upper anterior chest wall (Pedro José Labronici, et al. rev bras ortop. 2013; 48(4):317-321.), which is a common occurrence following such types of bone plate removal procedures. The numbness improves in the vast majority, but up to commonly persists to some degree for 2 years, and may be permanent and underreported in the literature. Moreover, the development of painful neuromas after iatrogenic transsection of the supraclavicular nerve after the bone plate removal has been described.

There has been no method to protect the supraclavicular nerve after clavicle fracture fixed with bone plates which are designed to be removed later.

### BRIEF SUMMARY OF THE INVENTION

It is an objective of the invention to provide a three-dimensional fracture fixation device for treatment of clavicle fracture, which permits protection of the supraclavicular nerve during removal of the fracture fixation device after clavicle fracture has healed.

The essential components of the three-dimensional fracture fixation device according to the invention include at least one fixation locking plate, a nerve protection scutum (shield), junctional hooks connecting the fixation locking plate and supporting the nerve protection scutum (shield), and bone screws, which are assembled to form a defined space to accommodate the supraclavicular nerve during open reduction and internal fixation.

The invention also provides a method for treating clavicle fracture with nerve protection, comprising: identifying the supraclavicular nerves around or close to the clavicle fracture or fractures; affixing at least one first bone plate (the fixation locking plate) to the aligned bone segments of the fractured clavicle, so that the first bone plate extends across the clavicle fracture or fractures; positioning one or more junctional hooks protruding above a side, edge or surface of the first bone plate, wherein a plurality of holes are extended through the first bone plate and the junctional hooks and designed to insert fixed-angle/angular-stable bone screws or pegs; positioning a second bone plate (the nerve protection scutum (shield)) with holes extending therethrough to rest upon the top surfaces of the junctional hooks, and securing the second bone plate to the junctional hooks by bone screws or pegs via the holes of the second bone plate and the junctional hooks, thus providing an area defining a space formed between the clavicle, the junctional hooks, the first bone plate and the second bone plate to accommodate the identified supraclavicular nerves, and thus preventing the supraclavicular nerves injury from later iatrogenic transsection wherein the first bone plate is designed to be removed.

### BRIEF DESCRIPTION OF THE DRAWINGS

The invention can be more fully understood by reading the subsequent detailed description and examples with references made to the accompanying drawings, wherein:
FIG. 1 illustrates a three-dimensional fracture fixation device according to an exemplary embodiment of the invention for complex fractures involving the middle third and lateral third segments of clavicle and acromioclavicular joint with the intent to protect the supraclavicular nerve, wherein the bone plate assembly is designed to be removed after fractures have healed;
FIGS. 2A is an anterior view of a three-dimensional fracture fixation device for nerve protection according to another exemplary embodiment of the invention in case of fracture at the middle third segment of the clavicle;
FIG. 2B is a superior view of the three-dimensional fracture fixation device shown in FIG. 2A;
FIG. 2C is a cross-sectional view taken along the line A-A' in FIG. 2B;
FIG. 3A is an anterior view of a three-dimensional fracture fixation device for nerve protection according to yet another exemplary embodiment of the invention in case of fracture at the lateral third segment of the clavicle;
FIG. 3B is a superior view of the three-dimensional fracture fixation device shown in FIG. 3A;
FIGS. 3C and 3D are cross-sectional views taken along the lines B-B' and C-C' in FIG. 3B.

### DETAILED DESCRIPTION OF THE INVENTION

The invention relates to a three-dimensional fracture fixation device for treating clavicle fracture with the intent to protect the supraclavicular nerve from later iatrogenic transsection, wherein the fracture fixation device is designed to be removed.

For example, and without limitation, the three-dimensional fracture fixation device according to the invention may be used for treatment of fractures of the lateral third, middle third or/and medial third segments of the clavicle.

The invention may be further understood with reference to the following description and the appended drawings, wherein like elements are referred to with the same reference numerals.

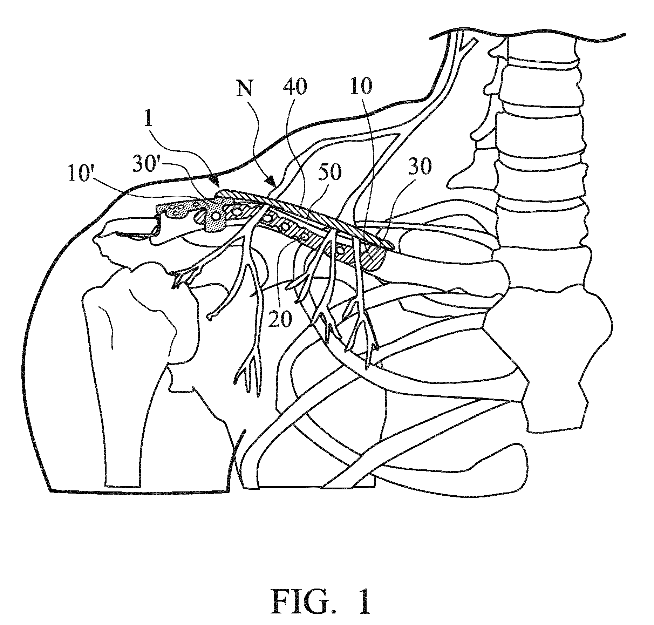

Referring to FIG. 1, the supraclavicular nerve N, originating from the C3 and C4 nerve roots and dividing into three primary branches, supplies innervation of the upper anterior chest wall. Also, the medial and intermediate branch groups are depicted in relationship to a three-dimensional fracture fixation device (bone plate assembly) 1 according to an exemplary embodiment of the invention.

As shown in FIG. 1, the three-dimensional fracture fixation device 1 includes an anterior fixation locking plate 10 with a junctional hook 30 extended upwardly therefrom, a distal fixation locking plate 10' with another junctional hook 30' extended upwardly therefrom, bone fixation elements such as bone screws or pegs 20, and a nerve protection scutum (shield) 40 above the junctional hooks 30 and 30'. It should be appreciated that the three-dimensional fracture fixation device 1 provides 1) a single solution for fixation of multiple complex middle and lateral clavicle fractures with acromioclavicular joint injuries, 2) a space 50 to accommodate the supraclavicular nerve N to prevent entrapment, and 3) a protection of the supraclavicular nerve N from cutting injury at the time of incision when the bone plate assembly is going to be removed.

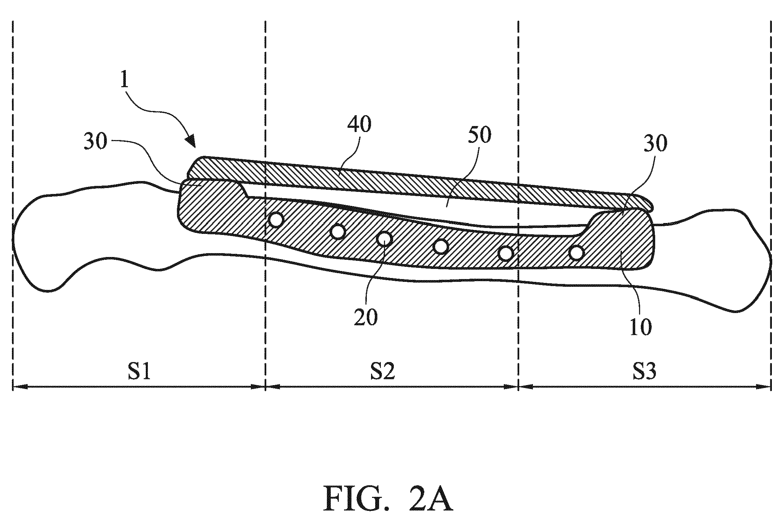

Next, referring to FIGS. 2A to 2C, a three-dimensional fracture fixation device 1 according to another exemplary embodiment of the invention for treating fracture of the middle third segment S2 (between the lateral third segment S1 and the medial third segment S3) of the clavicle is provided, including a fixation locking plate 10, bone fixation elements such as bone screws or pegs 20, two junctional hooks 30, and a nerve protection scutum (shield) 40.

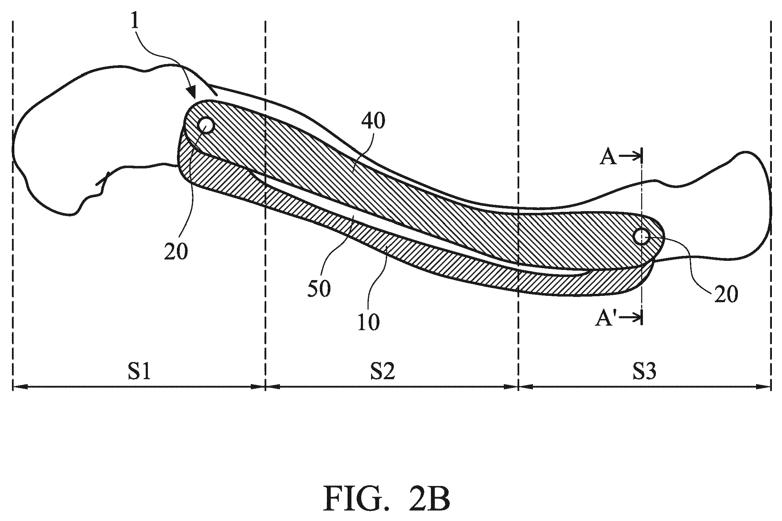

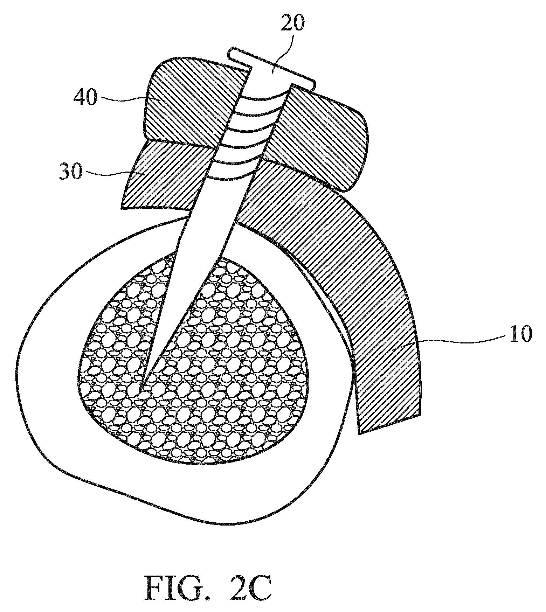

The fixation locking plate 10 is sized and shaped to be attached to the anterior surface of the middle third segment S2 of the clavicle via the bone screws 20, and the two junctional hooks 30 are extended upwardly from the fixation locking plate 10 and hung onto the superior surface of the clavicle. In this embodiment, each of the junctional hooks 30 is fixed within a portion, such as an end of the fixation locking plate 10. Further, the nerve protection scutum (shield) 40 is disposed upon the top surfaces of the two junctional hooks 30 and secured with the junctional hooks 30 via the bone screws 20 passing through the corresponding holes of the nerve protection scutum (shield) 40 and the junctional hooks 30 (FIG. 2C). From FIGS. 2B and 2C, the holes of the nerve protection scutum (shield) 40 are configured on a top surface thereof to receive heads of the bone screws 20. Thus, the clavicle, the first bone plate 10, the junctional hooks 30, and the second bone plate 40 form a defined space 50 therebetween to accommodate the supraclavicular nerve branches (not shown).

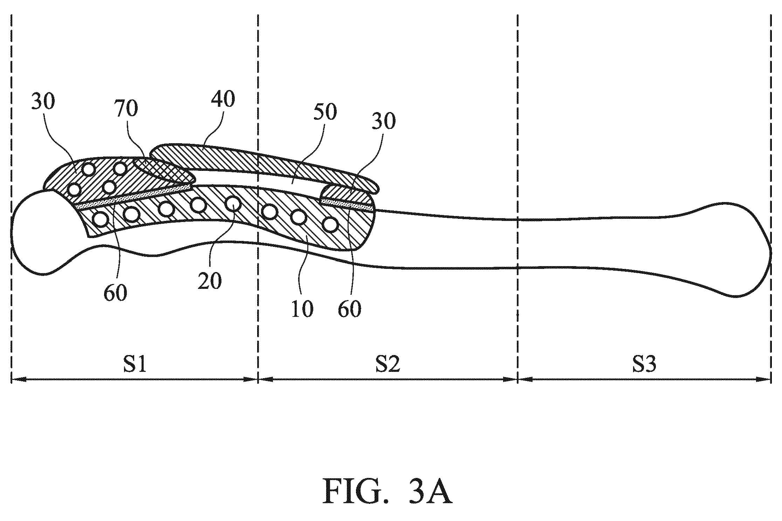

Next, referring to FIGS. 3A to 3D, a three-dimensional fracture fixation device 1 according to yet another exemplary embodiment of the invention for treating fracture of the lateral third segment S1 of the clavicle is provided, including a fixation locking plate 10, bone fixation elements such as bone screws or pegs 20, two junctional hooks 30, a nerve protection scutum (shield) 40, two fastening elements or linkers 60, and a cap or coating 70.

The fixation locking plate 10 is sized and shaped to be attached to the anterior surface of the lateral third segment S1 of the clavicle via the bone screws 20. One junctional hook 30 (the left-side one in FIG. 3A) with a scalloped top surface and another junctional hook 30 (the right-side one in FIG. 3A) are separated from the fixation locking plate 10. Also, the junctional hooks 30 have connecting portions coupled to the fixation locking plate 10 via the two fastening elements or linkers 60 and are hung onto the superior surface of the clavicle. In this embodiment, one of the junctional hooks 30 is treated with a cap or coating 70 (FIG. 3A), but the two junctional hooks 30 may also be treated with caps or coatings 70.

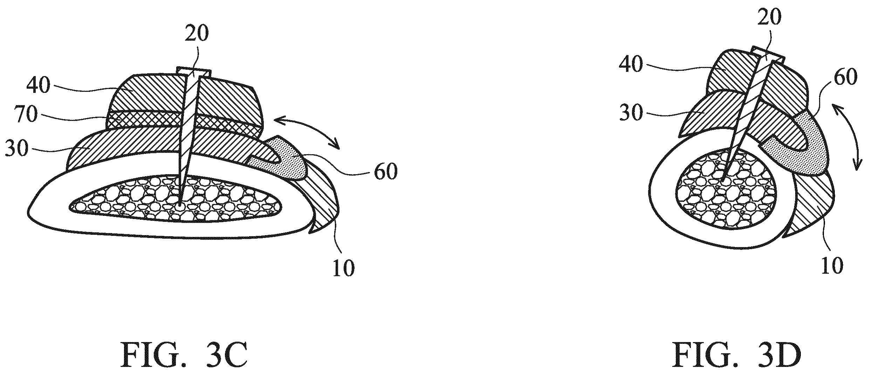

Further, the nerve protection scutum (shield) 40 is disposed upon the top surfaces of the two junctional hooks 30 and secured with the junctional hooks 30 via the bone screws 20 passing through the corresponding holes of the nerve protection scutum (shield) 40 and the junctional hooks 30 (FIGS. 3C and 3D). Thus, the clavicle, the first bone plate 10, the junctional hooks 30, and the second bone plate 40 also form a defined space 50 therebetween to accommodate the supraclavicular nerve branches (not shown).

The embodiment of FIGS. 3A to 3D differs from the embodiment of FIGS. 2A to 2C in that the cap or coating 70 is added on the top surface of the junctional hook 30 to increase the space 50 for supraclavicular nerve passing. Specifically, the junctional hook 30 has a raised section with a thickness of between, for example, 0.1 mm to 10 mm, wherein the thickness is increased by adding the cap or coating 70 on the junctional hook 30.

Moreover, the fastening elements or linkers 60 in the embodiment of FIGS. 3A to 3D permit in situ attachment and/or detachment of the junctional hooks 30 to the fixation locking plate 10 and adjustment of the angles and relative positions between the junctional hooks 30 and the fixation locking plate 10 to fit the clavicle contoured surface.

For example, the fastening element or linker 60, when in a first position, permits the junctional hook 30 to be coupled to the fixation locking plate 10. When the fastening element or linker 60 is in a second position, the junctional hook 30 is coupled to the fixation locking plate 10 such that the junctional hook 30 is rotatable relative to the fixation locking plate 10 about a rotation axis which extends along a longitudinal-axis direction of the fastening element or linker 60 (as the arrows indicate in FIGS. 3C and 3D) within a predetermined range of angulation. When the fastening element or linker 60 is in a third position, the junctional hook 30 is fixed in a desired position relative to the fixation locking plate 10 via, for example, a friction fit. Thus, the junctional hook 30 may be rotatable relative to the fixation locking plate 10 at an angle ranging from between, for example, 0 and 120 degrees in an antero-posterior direction, so that the surgeon may place the junctional hook 30 in a position in which the junctional hook 30 has maximum contact with the clavicle contoured surface. The top surface of the junctional hook 30 then provides a support for placing the nerve protection scutum (shield) 40.

The junctional hook 30 is also bendable to fit the clavicle contoured surface. For example, the junctional hook 30 may have an angle of bending which varies between, for example, 30 and 150 degrees.

In some embodiments, the junctional hook 30 is C-shaped, L-shaped, S-shaped, T-shaped, O-shaped, P-shaped, or ring-shaped.

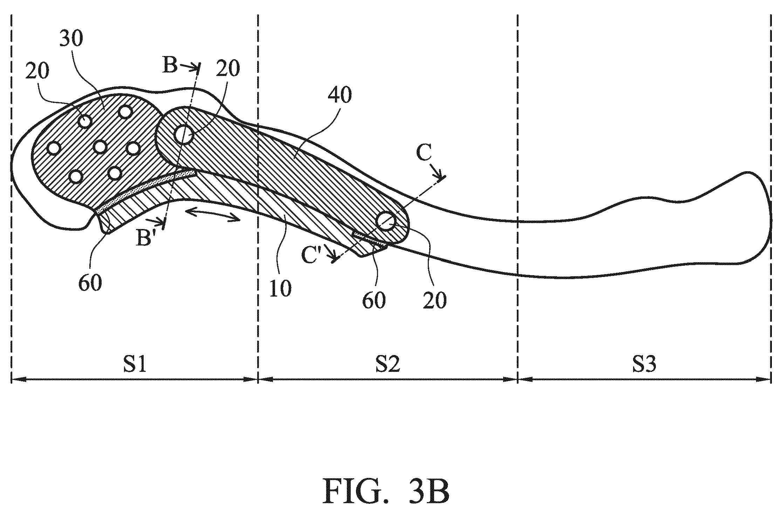

In some embodiments, the junctional hook 30 connecting the fastening element or linker 60 is also movable relative to the fixation locking plate 10 along a longitudinal-axis direction of the fixation locking plate 10 (as the arrow indicates in FIG. 3B).

In some embodiments, the junctional hook 30 includes holes in a part of the junctional hook 30 overlapping the clavicle. Thus, the bone screws 20 may be inserted through the holes of the junctional hook 30 to reinforce the fixation of the junctional hook 30 as well as the fixation locking plate 10 to the clavicle (FIG. 3B).

In some embodiments, the holes of the junctional hooks 30, the fixation locking plate 10 and the nerve protection scutum (shield) 40 are sized and shaped to receive the bone screws or pegs 20 therethrough.

In some embodiments, the fixation locking plate 10 and the nerve protection scutum (shield) 40 has outriggers, jigs, blunted ends, or scalloped ends on sides, surface, ends, edges thereof and/or around the plate bodies thereof. In some embodiments, the fixation locking plate 10 and the nerve protection scutum (shield) 40 may be flat or three-dimensionally curved.

The present invention also provides a method for treating clavicle fracture with nerve protection, including: identifying the supraclavicular nerves (the supraclavicular nerve N in FIG. 1) around or close to the clavicle fracture or fractures; affixing at least one first bone plate (the fixation locking plates 10, 10' in FIGS. 1 to 3D) to the aligned bone segments of the fractured clavicle, so that the first bone plate extends across the clavicle fracture or fractures; positioning one or more junctional hooks (the junctional hooks 30, 30' in FIGS. 1 to 3D) protruding above a side, edge or surface of the first bone plate, wherein a plurality of holes are extended through the first bone plate and the junctional hooks and designed to insert fixed-angle/angular-stable bone screws or pegs; positioning a second bone plate (the nerve protection scutum (shield) 40 in FIGS. 1 to 3D) with holes extending therethrough to rest upon the top surfaces of the junctional hooks, and securing the second bone plate to the junctional hooks by bone screws or pegs via the holes of the second bone plate and the junctional hooks, thus providing an area defining a space (the space 50 in FIGS. 1, 2A and 3A) formed between the clavicle, the junctional hooks, the first bone plate and the second bone plate to accommodate the identified supraclavicular nerves, and thus preventing the supraclavicular nerves injury from later iatrogenic transsection wherein the first bone plate is designed to be removed of the fractured clavicle.

The method further includes the steps of: reducing one of the clavicle fracture and the dislocation of the bone segments by the first bone plate, and selecting one or more junctional hooks having a desired hook size, shape and angle; coupling the selected junctional hooks with the first bone plate via fastening elements or linkers, and positioning, rotating or moving the junctional hooks along the first bone plate onto the clavicle; placing the second bone plate on the top surfaces of the junctional hooks that connect the first bone plate, with or without additional caps or coatings on the junctional hooks to create or increase a space formed between the first bone plate, the second bone plate, the junctional hooks and the clavicle, wherein the space is large enough to accommodate the supraclavicular nerves; and securing and affixing the second bone plate to the junctional hooks by the bone screws or pegs via the holes on the second bone plate and the junctional hook.

One of the advantages of the present invention is that the first bone plate (the fixation locking plate), the junctional hooks and the second bone plate (the nerve protection scutum (shield)) can be assembled in situ to create or increase a space to accommodate the supraclavicular nerves during open reduction and internal fixation for clavicle fracture. Another advantage of the present invention is that after months or years the bone plates and the junctional hooks can be disassembled in situ and removed from the patient while minimizing the risk of iatrogenic transsection of the supraclavicular nerves during skin incision and tissue dissection.

While the invention has been described by way of example and in terms of the preferred embodiments, it is to be understood that the invention is not limited to the disclosed embodiments. On the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A method for treating clavicle fracture with nerve protection, comprising:
identifying supraclavicular nerves around or close to the clavicle fracture or fractures;
affixing at least one first bone plate to aligned bone segments of the fractured clavicle, so that the first bone plate extends across the clavicle fracture or fractures;
positioning one or more junctional hooks protruding above a side, edge or surface of the first bone plate, wherein a plurality of holes are extended through the first bone plate and the junctional hooks and designed to insert bone screws or pegs;
positioning a second bone plate with holes extending therethrough to rest upon top surfaces of the junctional hooks, and securing the second bone plate to the junctional hooks by bone screws or pegs via the holes of the second bone plate and the junctional hooks, thus providing an area defining a space formed between the clavicle, the junctional hooks, the first bone plate and the second bone plate to accommodate the identified supraclavicular nerves, and thus preventing the supraclavicular nerves injury from later iatrogenic transsection wherein the first bone plate is designed to be removed.

2. The method for treating clavicle fracture with nerve protection as claimed in claim 1, wherein the holes of the second bone plate are configured on a top surface thereof to receive heads of the bone screws or pegs.

3. The method for treating clavicle fracture with nerve protection as claimed in claim 1 or 2, wherein the junctional hook is extended from the first bone plate.

4. The method for treating clavicle fracture with nerve protection as claimed in claim 3, wherein the junctional hook is fixed within a portion of the first bone plate.

5. The method for treating clavicle fracture with nerve protection as claimed in claim 1 or 2, wherein the junctional hook includes a connecting portion connecting the first bone plate.

6. The method for treating clavicle fracture with nerve protection as claimed in claim 5, further comprising:
providing one or more fastening elements or linkers to couple the connecting portion of the junctional hooks to the first bone plate.

7. The method for treating clavicle fracture with nerve protection as claimed in claim 6, wherein the junctional hook connecting the fastening element or linker is movable relative to the first bone plate along a longitudinal-axis direction of the first bone plate.

8. The method for treating clavicle fracture with nerve protection as claimed in claim 6, wherein the junctional hook is rotatable relative to the first bone plate about a rotation axis which extends along a longitudinal-axis direction of the fastening element or linker.

9. The method for treating clavicle fracture with nerve protection as claimed in any of claims 1 to 8, further comprising:
providing a cap or coating on the junctional hook to increase the space for accommodating the supraclavicular nerves.

10. The method for treating clavicle fracture with nerve protection as claimed in any of claims 1 to 9, wherein the junctional hook is one of C-shaped, L-shaped, S-shaped, T-shaped, O-shaped, P-shaped and ring-shaped.

11. The method for treating clavicle fracture with nerve protection as claimed in any of claims 1 to 10, wherein the first bone plate has outriggers, jigs, blunted ends, or scalloped ends on sides, surface, ends, edges thereof and/or around a plate body thereof.

12. The method for treating clavicle fracture with nerve protection as claimed in any of claims 1 to 11, wherein the second bone plate has outriggers, jigs, blunted ends, or scalloped ends on sides, surface, ends, edges thereof and/or around a plate body thereof.

13. The method for treating clavicle fracture with nerve protection as claimed in any of claims 1 to 12, wherein the holes of the junctional hooks, the first bone plate and the second bone plate are sized and shaped to receive the bone screws or pegs therethrough.

14. The method for treating clavicle fracture with nerve protection as claimed in any of claims 1 to 13, wherein the first and second bone plates are flat or three-dimensionally curved.

15. The method for treating clavicle fracture with nerve protection as claimed in any of claims 1 to 14, further comprising:
reducing one of the clavicle fracture and a dislocation of the bone segments by the first bone plate, and selecting one or more junctional hooks having a desired hook size, shape and angle;
coupling the selected junctional hooks with the first bone plate via fastening elements or linkers, and positioning, rotating or moving the junctional hooks along the first bone plate onto the clavicle;
placing the second bone plate on the top surfaces of the junctional hooks that connect the first bone plate, with or without additional caps or coatings on the junctional hooks to create or increase the space formed between the first bone plate, the second bone plate, the junctional hooks and the clavicle, wherein the space is large enough to accommodate the supraclavicular nerves; and
securing and affixing the second bone plate to the junctional hooks by the bone screws or pegs via the holes on the second bone plate and the junctional hook.
